# EUROPEAN PATENT SPECIFICATION

(11) **EP 1 968 689 B1**
(45) Date of publication and mention of the grant of the patent: **06.06.2012**
(21) Application number: 06846070.8
(22) Date of filing: 27.12.2006
(51) Int. Cl.: A61M 31/00, A61M 5/158, A61M 25/06

(54) **PORTAL ACCESS DEVICE WITH REMOVABLE SHARPS PROTECTION**
PORTALE ZUGANGSVORRICHTUNG MIT ENTFERNBAREM SICHERHEITSSCHUTZ
DISPOSITIF D'ACCES A UN ORIFICE COMPRENANT UNE PROTECTION AMOVIBLE CONTRE DES OBJETS POINTUS ET TRANCHANTS

(30) Priority: 28.12.2005 US 318484
(43) Date of publication of application: 17.09.2008
(73) Proprietor: Smiths Medical ASD, Inc., St. Paul, MN 55112 (US)
(72) Inventor: MICHELS, Lester, D., Eden Prairie, MN 55347 (US); BELING, William L., Newbrighton, Minnesota 55112 (US); TRAVIS, Ronald G., Spring Lake Park, Minnesota 55432 (US)
(74) Representative: Lerwill, John
(86) International application number: PCT/US2006/049357
(87) International publication number: WO 2007/079114

(56) References cited:
- US-A- 5 545 143
- US-A- 5 858 005
- US-B1- 6 719 721
- US-B2- 6 613 015

## Description

### Field of the Invention

The present invention relates to a device for accessing a portal reservoir, and more particularly a portal access device that combines long term accessing of a portal reservoir with sharps protection.

### Background of the Invention

Long term intravenous therapy to a patient oftentimes requires that a portal reservoir (also may be referred herein as port or portal) be implanted to the patient. The medicament stored in the port is fed slowly to the patient via a catheter connected to the portal. To refill the portal, conventionally a needle is inserted through the skin of the patient into a septum of the portal so that the portal may be refilled with the desired medicament. As with the use of any needle or sharps instruments, there is always a chance that the user, or a bystander, may be accidentally pricked by the contaminated needle when it is removed from the patient. Such accidental injury may well be serious as the tip of the needle is contaminated with the fluid from the patient.

A prior device used for shielding the contaminated tip of the needle is disclosed in U.S. patent 6,613,015, assigned to the assignee of the instant application and marketed under the trade name GRIPPER PLUS. In the '015 patent, a right angle needle for accessing the portal is disclosed. The '015 device has the horizontal portion of its right angle needle embedded in an arm that has a distal portion hingedly connected to uprights of a base. The vertical portion of the needle extends through an opening at the proximal portion of the base. Medicament or fluid is fed to the right angle needle via a catheter connected to the distal end of the horizontal portion of the needle. A well or catch is formed above the main body of the base adjacent the opening so that when the arm is pivotally raised relative to the base and when the needle is fully pulled out of the base, the tip of the needle would flex forward so as to be entrapped in the catch adjacent to the opening. Even though surrounded by a dam, the tip of the needle nonetheless is exposed to the environment and viewable by the patient, and there is a remote chance that contaminated fluid at the tip of the needle may adherer to the well, or may be splattered unnecessarily around the area of the wall or beyond. A similar right angle device is disclosed in U.S. patent 6,719,721.
After accessing the portion and refilling it, the device of the '015 patent is removed from the skin of the patient. Any subsequent refilling of the portal requires that a new right angle needle device be used to gain access to the portal. This repeated insertion of a needle to the patent tends to cause discomfort, at the very least, to the patient.
There is therefore a need for a portal access device that may be secured to the patient on a long term basis so as not to subject the patient to repeated inserts by needle sharps.
There is also a need for a sharps protection device having the tip of its needle that, once removed from the patient, will not be exposed to the environment or seen by the patient.
U.S. 5545143, which is considered to represent the closest prior art, discloses a device for subcutaneous medication delivery in which a body has a soft cannula extended downwards from a generally flat surface and a self sealing septum mounted on the centre of a top surface is which is generally of concave shape sloping downwards towards it outer perimeter, at which point the single body is very thin. A needle is caused to penetrate through the septum and through the lumen of the soft cannula used for inserting the cannula through the skin. Once the soft cannula is placed subcutaneously, the needle is removed and adhesive tape placed over the single body and on to the skin beyond the patient's outer perimeter.
Another prior art configuration is known from US 5858005.
According to the first aspect of the present invention there is provided an apparatus comprising: an infuser having a body of a given shape, a blunt cannula extending downwardly from a lower surface of the body and a tube connected to the body for establishing a fluid communication path between said tube and said blunt cannula; a base having a distal portion and a proximal portion, having a configuration that form fits over the given shape of the body of said infuser, an arm having a second end hingedly and movably connected to the distal portion of said base, a sharp cannula attached to a first end of said arm, a bore defined between an opening at an upper surface and another opening at a lower surface at the proximal portion of said base, the proximal portion of said base form fitting over the body of said infuser when said sharp cannula is removably mated to said blunt cannula through said bore at the proximal portion of said base and the tip of said sharp cannula exposed beyond the tip of said blunt cannula with said arm lying substantially longitudinally along said base; wherein said sharp cannula is removed from said blunt cannula when said first end of said arm is moved pivotally in a direction away from said base.
The present invention further provides a method of establishing a fluid path between a fluid store and a port implanted in a patient, comprising the steps of: (a) providing an infuser having a body of a given shape and a blunt cannula extending downwardly therefrom and a tube having one end connected thereto and another end connected to said fluid store, said tube being in fluid communication with said blunt cannula; (b) providing a base having a sharp cannula and a proximal portion having a configuration that has a shape that form fits over the body of said infuser; (c) form fitting the proximal portion of said base over the body of said infuser and removably mating said sharp cannula to said blunt cannula until the tip of said sharp cannula is exposed beyond the tip of said blunt cannula; (d) inserting the combined sharp and blunt cannulas to the patient to position said cannulas into said port; and (e) removing said sharp cannula from said blunt cannula while leaving said blunt cannula in said port; whereby a fluid communication path is established between said port and said fluid store through said blunt cannula and said infuser.

The main body of the infuser, or its cap or housing, is the portion of the component external to the patient and is meant to be positioned on or above the patient's skin surface, along with its attached tubing. The blunt cannula is attached to and extends downwardly from the infuser housing through the skin of the patient and the portal septum after placement, and establishes the fluid communication path between the external tubing of the infuser assembly and the portal reservoir implanted in the patient.

To be properly inserted to the patient, the needle sharps device is superposed, or fits, over the infuser in such a way that its needle or sharp cannula extends past the self sealing septum of the infuser, through the infuser, and axially into the blunt cannula, with the tip of the sharp cannula protruding or exposed from the end of the blunt cannula. The sharp cannula thereby serves as the means to puncture and penetrate first the patient and then the self sealing septum at the portal reservoir to provide a track for the coaxial blunt cannula. Once the sharp cannula and the blunt cannula, or at least corresponding portions of the respective tips thereof, are coaxially positioned correctly within the portal reservoir, the sharp cannula is removed from the blunt cannula. Once the sharp cannula is removed from the infuser assembly, the safety needle protection device may be removed from the infuser assembly. The tip of the needle or sharp cannula is fixedly maintained within a bore of the base of the needle insertion device that extends between the opening at the top surface and the opening at the lower surface of the base wherethrough the sharp cannula extends into the blunt cannula.

Respective locking mechanisms are provided at the end of the arm of the needle insertion device that connects to the base and the uprights at the base to which the arm is hingedly or pivotally connected to prevent the arm from further movement, be it upwards or downwards, relative to the base once the tip of the needle is positioned within the bore of the base. The sharps protection device may then be safely and properly discarded.

The infuser assembly remains on the patient, with the septum of the infuser where the sharp cannula entered and exited self-sealed. With proper attachment to the patient, either adhesively or by tape, the low profile infuser assembly provides long term access to the portal reservoir without further risks of sharps injury, or further unnecessary pain and discomfort, to the patient due to the need for reinsertions of sharp needles to the patient

To hide the contaminated tip of the needle or the sharp cannula from the user and also the patient, the needle insertion device of the instant invention is configured such that when the tip of the contaminated needle is withdrawn first from the portal reservoir and then the patient, as soon as it is positioned appropriately in the bore formed in the base through which the sharp needle or cannula extends, the movement of the arm that bears the sharp cannula is stopped and locked into place, so that the tip of the needle would not come into contact with any part of the base, and is not withdrawn above the top surface, or the opening at the top surface, of the base. Locking mechanisms at the end of the arm that hingedly connects to the base and at the uprights to which the arm is hingedly attached cooperatively coact to prevent any further upward and downward movements of the arm, once the tip of the needle is appropriately positioned within the bore.

The lower surface of the base of the needle insertion device is configured to form fittingly mount over the infuser, both the infuser or cap portion and the tube connecting thereto that lie over the patient's skin surface, so that the needle injection device and the infuser may be pre-assembled as a unit.

The needle insertion device of the instant invention is preferably a safety needle device that comprises a base having a distal portion and a proximal portion, an opening provided at the proximal portion of the base, a bore extending from the opening at the proximal portion of the base, an arm having a first end and a second end, the second end of the arm being hingedly connected to the distal portion of the base so that the arm is pivotally moveable relative to the base at its second end; and a right angle cannula mounted to the arm, a vertical portion of the cannula including the tip of the cannula extending downwardly from the first end of the arm and passes through the opening of the base before use, a horizontal portion of the cannula mounted along the length of the arm, so that when the first end of the arm is moved to its upmost position, the vertical portion of the cannula is withdrawn from the opening and the tip of the cannula is positioned within the bore.

The safety needle device of the instant invention may further include a first lock mechanism having a first and second part provided at the distal portion of the base, and a second lock mechanism having a first and second part provided at the second end of the arm with the respective first parts of the first and second lock mechanisms coacting to prevent further movement of the arm relative to the base along one direction after the tip of the cannula is positioned within the bore, and the respective second parts of the first and second lock mechanisms coacting to prevent further movement of the arm relative to the base along another direction once the tip of the cannula is positioned within the bore.

### Brief Description of the Figures

The present invention will become apparent and the invention itself will be best understood by reference to the following description of the present invention taken in conjunction with the accompanying drawings, wherein:
Fig. 1 is a perspective view of the safety needle inserted device of the instant invention;
Fig. 2 is a perspective view of a first embodiment of the safety needle inserted which does not fall within the scope of the instant invention;
Fig. 3 is an enlarged view of the interconnection between the base and the arm of the safety needle inserted of Fig. 2;
Fig. 4 is another view of the interconnection between the arm and the base of the safety needle inserted of Fig. 2;
Fig. 5 is another embodiment of the safety needle inserted which does not form part of the instant invention showing in particular the location of the tip of the needle, or sharp cannula, positioned within the bore of the base;
Fig. 6 is a side view of another embodiment of the safety needle inserter which does not form part of the instant invention, with the arm of the inserter shown lying longitudinally along the base and the sharp cannula extending downwardly from the base;
Fig. 7 is another view of the safety needle inserter of the Fig. 6 embodiment showing the sharp cannula having been withdrawn from the opening of the base with the tip residing within the bore of the base and the arm having been pivoted upwardly relative to the base;
Fig. 8 is a front perspective view of the safety needle inserter of Figs. 6 and 7, sans the sharp cannula, for showing the interrelationship between the locking mechanisms at the uprights of the base and the end of the arm hingedly connected to the uprights of the base;

Fig. 9 shows a portal access device of the instant invention in which the safety needle inserter component of the instant invention is superposed over an infuser assembly component of the instant invention;

Fig. 10 is a bottom view of the portal access device of the instant invention showing in particular the underside of the infuser and the safety needle inserter_{;}

Fig. 11a and Fig. 11b show the sharp cannula of the safety needle inserter mated axially to the blunt cannula of the infuser and withdrawn from the infuser, respectively; and

Fig. 12a and Fig. 12b are respective views of the portal access device showing the mating of the sharp cannula to the blunt cannula and the removal of the sharp cannula from the blunt cannula, respectively.

### Detailed Description of the Invention

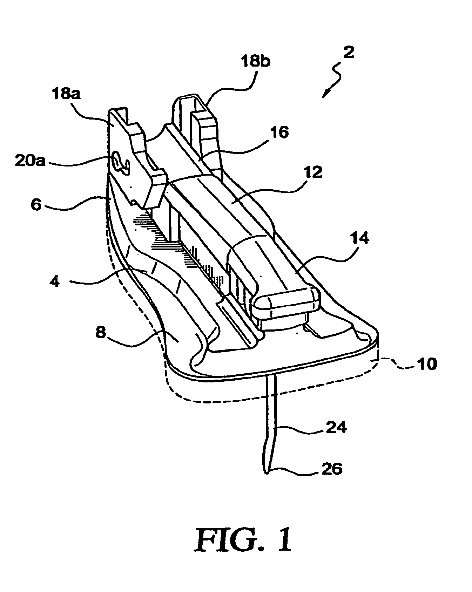

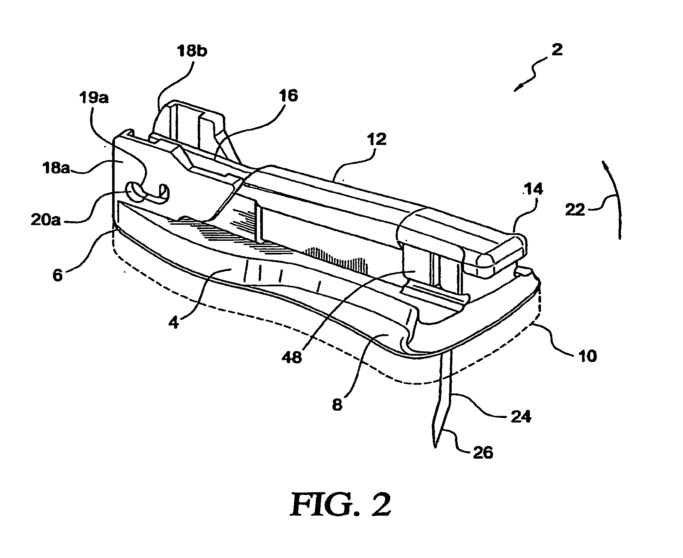

A first embodiment of the safety needle inserter device component which does not form part of the instant Invention is shown in Figs. 1-4. As shown, the safety needle inserter 2 has a base 4 that has a distal portion 6 and a proximal portion 8. A foam pad or layer 10 coated with an adhesive for adhering base 4 to the patient may be mounted to the underside, or the lower surface, of base 4. An arm having a first end 14 and a second end 16 is hingedly connected to two uprights 18a and 18b, respectively, at distal portion 6 of base 4. Two lugs 19, one at each side of second end 16 of arm 12, are movably journaled to the respective guide holes 20a and 20b of the respective uprights 18a and 18b, to thereby hingedly and movably connect arm 12 to base 4. Only lug 19a is shown in Fig. 2. When uplifted in the direction as indicated by directional arrow 22, arm 12 is pivotally moved, via its second end. 16, in an upward direction relative to base 4.

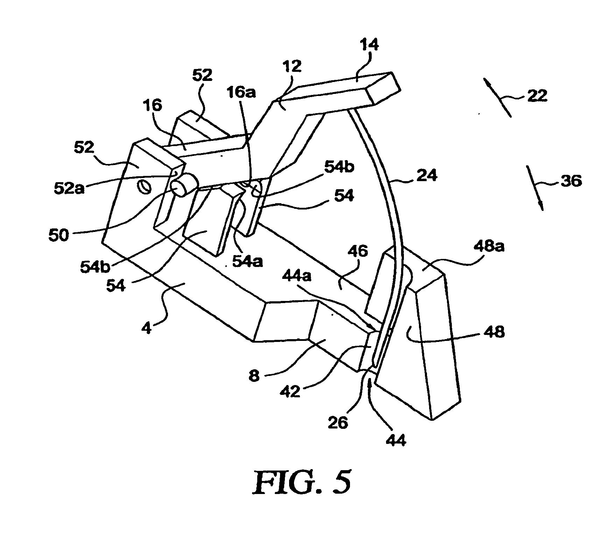

A needle, or sharp cannula 24, is mounted to the underside of arm 12, at its first end 14. As shown in Figs. 1 and 2, when arm 12 lies substantially longitudinally along the length of base 4, needle 24 extends downwardly from base 4 through an opening 44, best shown in the embodiment of Fig. 5. As shown, sharp cannula 24 has a tip 26 that is adapted to insert into the patient and penetrate the septum of a portal reservoir, or port, implanted to a patient. Note that even though the sharp cannula 24 of the safety needle inserter of the Figs. 1-4 embodiment is a unitary needle mounted vertically from arm 12, a right angle needle that has a vertical portion and a horizontal portion embedded in arm 12 may also be used as the safety needle inserter of the instant invention. In the case where a right angle needle is used, the distal end of the horizontal portion of the right angle needle may be connected to a catheter, tube or tubing so that fluid may be conveyed to, or removed from, the sharp cannula 24. The sharp cannula may be usable singly, or coaxially with a blunt cannula of the to be discussed infuser component of the instant invention.

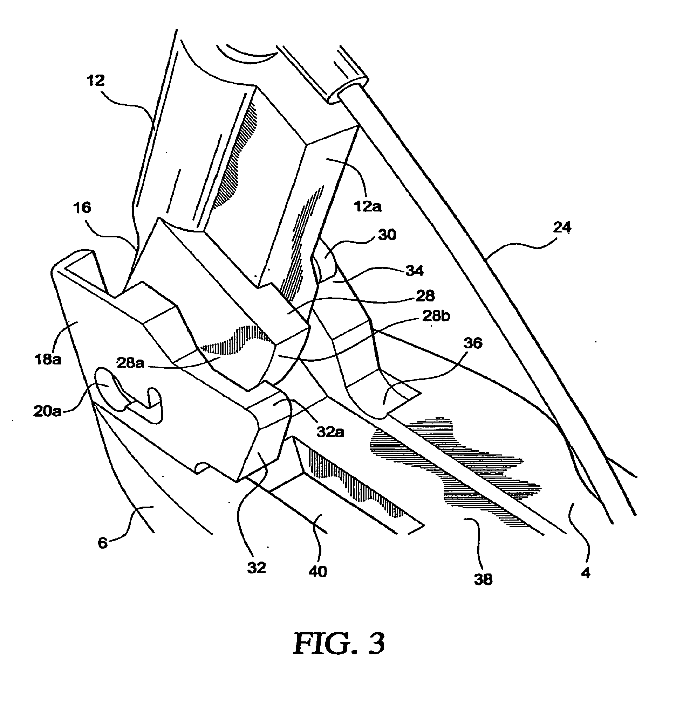

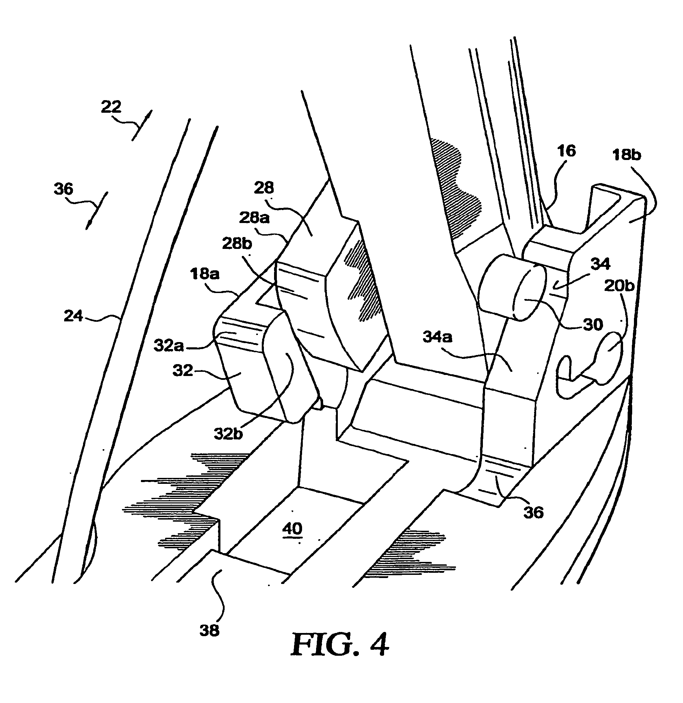

Figs. 3 and 4 illustrate the locking mechanisms at the respective uprights 18a and 18b at distal portion 6 of base 4 and the corresponding coacting locking mechanisms formed at end 16 of arm 12 for preventing further movement of arm 12, once it has been pivotally moved relative to base 4 when tip 26 of sharp cannula 24 is positioned within a bore 42 formed in base 4, best illustrated in the embodiment of the safety needle inserter shown in Fig. 5. Although disclosed as different lock mechanisms, the different portions at both arm 12 and uprights 18 of base 4 may be considered as different parts of a single locking mechanism that cooperatively work together to lock arm 12 in place relative to base 4, and more importantly to fixedly maintain tip 26 of sharp cannula 24 within bore 42 formed at proximal portion 8 of base 4, so as to prevent tip 26 from being exposed or fluid thereon from being splattered, when sharp cannula 24 is withdrawn from the patient.

In particular, the lock mechanisms, or the portions of the lock mechanism, as shown in the Fig. 3 and 4 embodiment, comprise a leg 28 and a boss 30 at the respective sides of end 16 of arm 12. The lock mechanisms, or the parts of the lock mechanism, at distal portion 6 of base 4 are configured as a shoulder 32 at upright 18a and a groove or recess 34 at upright 18b.

Due to the inherent elasticity in the medical plastics that are used for form molding the base and the arm of the safety needle insertion device, when arm 12 is moved along the direction indicated by directional arrow 22, the side surface 28a of leg . 28 would bias against the surface 32a of leg 32, as boss 30 may or may not be sliding along surface 34a of groove 34, so that once the foot 28b of leg 28 passes ledge 32b of shoulder 32, shoulder 32 will return to its original position so that latch 32b and foot 28b would blas or coact against each other to prevent any downward movement, as indicated by directional arrow 38, of arm 12. At substantially the same time, once boss 30 reaches groove 34 and biases thereagainst, further upward movement of arm 12 relative to base 4 is prevented.

To enable arm 12 to lie co-planarly on base 4, a notch 36 is provided on base 4 as a rest for boss 32. Further, a channel 38 is molded into base 4 to allow the downward extending portion 12a of arm 12 to fit in alignment to base 4, when arm 12 is in the position vis-a-vis base 4 as shown in Figs. 1 and 2. A void 40 is provided on base 4 to receive leg 28 of arm 12 so that arm 12 could lie substantially along base 4 as shown in Figs. 1 and 2.

Fig. 5 is an illustration of a second embodiment of the safety needle inserter which does not form part of the instant invention. Other than the overall look of the safety needle inserter and the various parts of the locking mechanisms which coact to lock arm 12 in place relative to base 4, the safety needle inserter of the Fig. 5 embodiment functions in the same way as the embodiment shown in Figs. 1-4. The components that are the same as those of the earlier disclosed embodiment are labeled the same.

As shown more clearly by the partial cut-away view, the tip 26 of sharp cannula 24, when withdrawn from the portal reservoir and the patient, is maintained within a bore 42 that is formed at the proximal portion 8 of base 4. As shown, bore 42 is defined between opening 44 at the underside or lower surface of base 4 and an opening 44a at the upper side or upper surface 46 of base 4. A dam 48 extends from proximal portion 8 of base 4 and serves as an additional safety feature to ensure that the tip 26 of cannula 24 could not be forcibly removed from bore 42. In addition, the top 48a of dam 48 provides a rest stop for arm 12. with end 14 thereof resting against top surface 48a, when needle 26 is axially fitted to the blunt cannula of the infuser. Dam 48 also provides a point for the needle inserter where a user can apply the force needed to insert the sharp cannula into the patient and, as will be described infra, penetrate the septum of the portal reservoir implanted to the patient. The same dam structure is provided in the earlier embodiment of the safety needle inserter.

The Fig. 5 embodiment of the safety needle inserter is different from the embodiment shown in Figs. 1-4 in that instead of using separate components for preventing upward and downward movements of arm 12, a pair of bosses 50 provided at the end 16 of arm 12 would bias against the respective front faces 52a of a pair of uprights 52 that are formed at the distal portion of base 4, to prevent further upward movement of arm 12 relative to base 4. A second pair of uprights 54 also extend from base 4 to grasp arm 12, when arm 12 lies longitudinally along base 4 with cannula 24 extending though opening 44. The holding of arm 12 by uprights 54 is by way of corresponding fingers 54a and 54b at the uprights 54. Since uprights 54, along with base 4 and arm 12, are all molded from medical plastics material, when arm 12 is moved upwardly per directional arrow 22, fingers 54b of the uprights 54 would give away until end portion 16 of arm 12 passes both fingers 54a and 54b. At which time uprights 54 would return to their respective original positions so that the lower surface 16a of end portion 16 of arm 12 would rest against the respective inclines 54b of uprights 54, thereby preventing arm 12 from any downward movement as indicated per directional arrow 36.

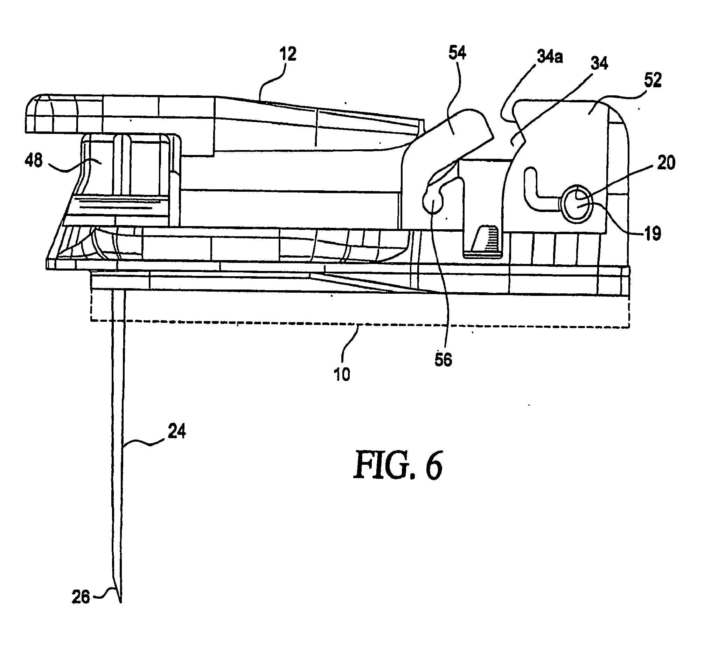

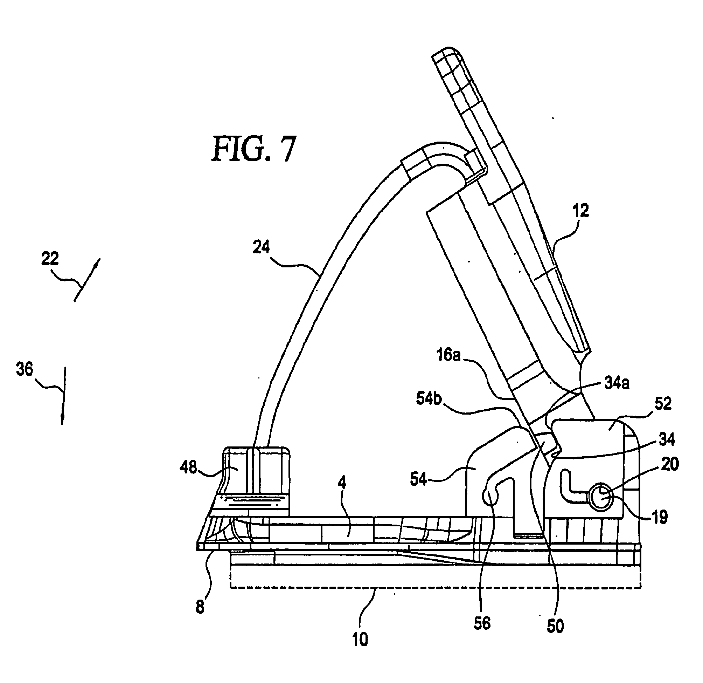

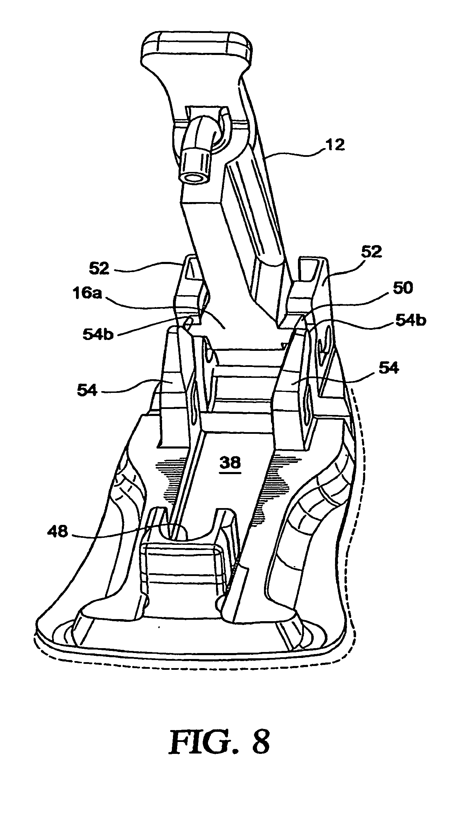

A third embodiment of the safety needle inserter which does not form part of the instant invention is shown in Figs. 6-8. This embodiment is a modification of the simplified embodiment shown in Fig. 5 in that uprights 54 have been reconfigured to allow additional flexibility by the adding of a notch 56 thereto. As a consequence, when arm 12 is pulled upwards along the direction as indicated by directional arrow 22, each of uprights 54 would tend to flex away, until the underside 16a of arm 12 clears the top edge 54b of the respective uprights 54. At which time, uprights 54 will return to their respective memorized original positions so as to move upper surfaces 54b of uprights 54 under the bottom surface 16a of arm 12 and more specifically those surfaces of the bosses 52, or areas proximate thereto, to thereby prevent downward movement of arm 12 along the direction as indicated by directional arrow 36.

Further, similar to the embodiment shown in Figs. 1-4, respective grooves or recesses 34 are provided at the uprights 52, so that bosses 52 are caught by the front surfaces of the respective recesses 34, designated by 34a, so as to prevent arm 12 from any further upward movement as indicated by directional arrow 22. As before, even though not shown for the sake of clarity, the tip of needle 24 is fixedly retained within a bore at proximal portion 8 of base 4. Note that Figs. 6-8 clearly show that arm 12 is hingedly connected to base 4 by way of its lugs 19 movably journaled to apertures 20 of uprights 52.

As with the other embodiments, a foam layer or pad 10 with an adhesive underside may be provided to the safety needle inserter for enhancing the comfort to the patient, when the inserter is used individually. However, as will be discussed *infra,* the fact that the safety needle inserter is a component of the overall portal access device of the instant invention, and its lower surface does not come into contact with the patient when used with the infuser component, foam 10 is not needed when the safety needle inserter is to be used with the infuser.

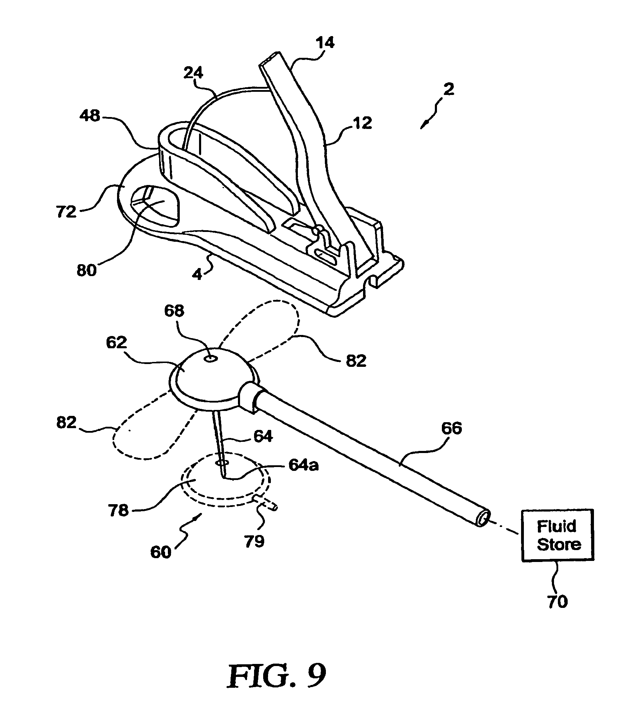

With reference to Figs. 9-12, the portal access device of the instant invention is shown. In particular, a safety needle inserter 2 as described above is used in conjunction with an infuser or a low-profile portal access assembly 60 that includes an infuser body or housing 62, a blunt cannula 64 attached to the underside of body 62 and a catheter or tube 66 that is attached to a side of body 62. As shown in Fig. 9, infuser body 62 is formed as a domed shaped housing or a manifold that has a self-sealing septum 68 at its top in axial alignment with blunt cannula 64. Given that housing 68 is substantially hollow, infuser 60 in essence provides a fluid storage depot through which a communication path between tube 66 and blunt cannula 64 is established.

In operation, tube 66 is connected to a fluid store 70, which may be a syringe or a pump or some other storage device that is adapted for storing fluids or medicaments to be infused to the patient. Alternatively, instead of infusing fluid to the patient, or more particularly to the portal reservoir which is punctured by blunt cannula 64, fluid in the portal reservoir may be retrieved by means of the fluid communication path established between blunt cannula 64 and tube 66 via infuser body 62.

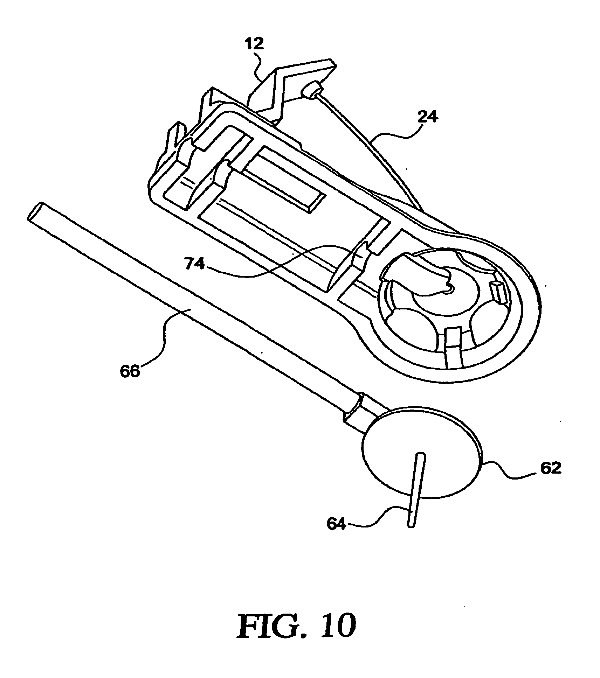

The safety needle inserter component of the portal access device of the instant invention, for the Fig. 9 embodiment, has its proximal portion configured as a dome 72 so that it may be form fitted over infuser body 62. Of course, infuser body 62 may have different dimensions and be configured as different shapes, for example a rectangular shaped housing. In which case, the proximal portion of base 4 of the safety needle inserter would also be rectangularly shaped so that it may form fit over the hypothetical rectangular shaped infuser body. Further, the remainder portion of base 4 may be configured to fit over tube 66, by having a channel 74 formed at its underside as shown by the bottom view of the safety needle inserter in Fig. 10.

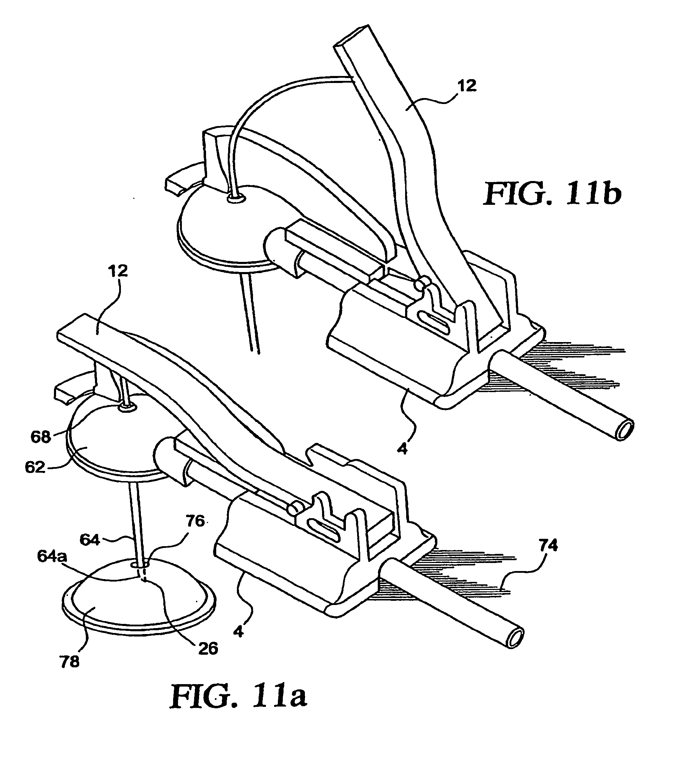

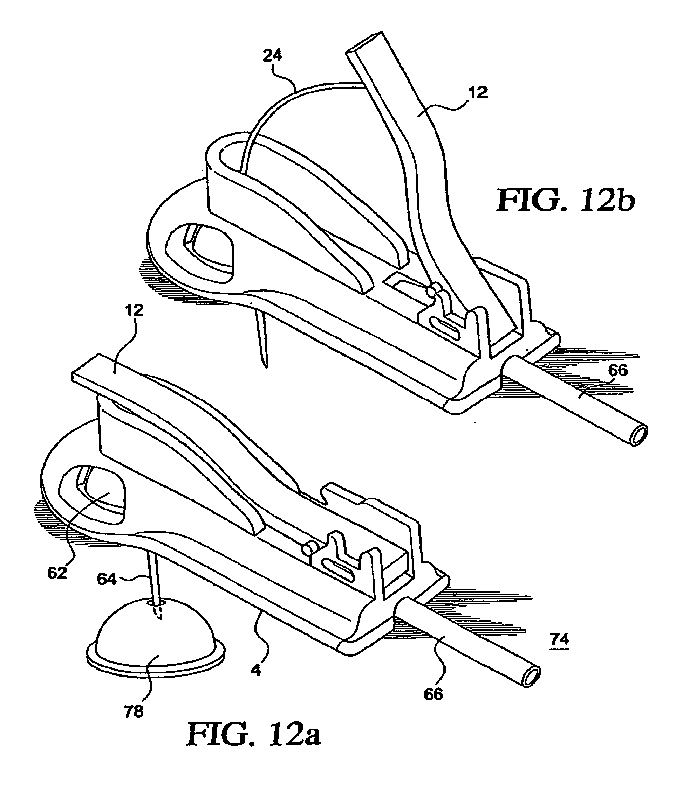

The operation of the safety needle inserter component of the portal access device as shown in Figs. 9 and 10 is the same as that discussed earlier with respect to the various embodiments of the safety needle inserter. When used with infuser assembly 60, safety needle inserter 2 is placed over infuser assembly 60 so that needle or sharp cannula 24 of the needle insertion device 2 is inserted through septum 68 into infuser body 62 and axially through blunt cannula 64 until the tip 26 of sharp cannula 24 protrudes or is exposed beyond the tip 64a of blunt cannula 64. By configuring the proximal portion of needle inserter 2 to form fit over the infuser housing 62, and with arm 12, in particular proximal portion 14 thereof, resting on dam 48 of base 4, a user can readily insert the combination blunt cannula 64/sharp cannula 24 into a patient, designated by 74 in Figs. 11 and 12, so that the tip 26 of cannula 24 may be inserted through a septum opening 36 of a port or portal reservoir 78 implanted in patient 74, per shown in Figs. 11a and 12a. A window 80 may be formed at the proximal portion 8 of base 4 to enable the user to confirm that the dome 72 of the safety needle inserter 2 is appropriately form fitted over infuser body 62, to thereby ensure that cannula 24 be inserted correctly into infuser body 62 and through blunt cannula 64.

Once it is determined that portal reservoir 78 has been properly punctured and connected to infuser body 62 by way of the concentric blunt/sharp cannulas, the safety needle inserter may be removed from the infuser assembly by lifting arm 12 of the needle inserter relative to its base 4, per shown in Figs. 11b and 12b. Once tip 24 of needle 24 is 'positioned within the bore of the base, with the respective lock mechanisms coacting at the arm and the base, arm 12 is prevented from further movement both upwardly and downwardly relative to base 4, as per discussion supra, so that tip 26 of needle 24 Is fixedly maintained or positioned within the bore 42 formed in base 4.

Once arm 12 is locked into place, with tip 26 of the needle 24 properly positioned within bore 42 of base 4, the safety needle inserter may be removed from infuser assembly 60. To ensure that Infuser body 62 and tube 66 would remain in place while needle inserter 2 is being removed, infuser body 62 may be provided with a pair of wings 82, either by bonding or molding, so that the user can hold wings 82 against the body or the skin surface of the patient, while lifting safety needle inserter 2 away from infuser assembly 60.

After removal of the safety needle inserter 2, infuser assembly 60 may be secured to the skin surface of the patient, by an adhesive layer preformed at the underside of infuser body 62 or an adhesive tape for example, so that a fluid communication path is continuously established between the portal reservoir 78 implanted in the patient and the fluid store 70. As a consequence, medicaments and other fluids are easily and readily infused to the patient through portal reservoir 78 and its associated catheter or tubing 79. Moreover, infuser assembly 60 provides a long term access to the portal reservoir 78 without further risks of sharps injury, as the safety needle inserter 2 has been removed and no further reinsertion is needed. Furthermore, given that the cannula 64 for infuser assembly 60 is a blunt cannula, when the infuser assembly is removed, there is no risk of injury to the patient or the user, and therefore no sharps protection is required.

Yet furthermore, given that septum 68 of the infuser assembly is self-sealing, infuser assembly 60 may be re-accessed through the self-sealing septum 68, either to provide direct coaxial access to the chamber of the portal reservoir 78, or access to the portal outlet catheter 79, for example by a radially flexible and axially stiff (pushable) wire or rod so that the fluid communication path may be threaded or rodded to clear any obstructions that may have occurred in either the portal reservoir or catheter 79 connected to the portal reservoir though which the medicament is fed to the patient.

Moreover, if a particular type of medicament is required, instead of providing the medicament through the fluid store 70, which feeds to tube 66 of the infuser assembly, a right angle needle insertion device such as that described earlier, or as disclosed in the above-discussed'015 patent, may be used to directly access the internal chamber of the portal reservoir, either via the blunt cannula of the infuser assembly or separate from the infuser assembly, so that the particular fluid may be fed through the catheter or tubing connected to the horizontal portion of the right angle needle directly into the portal reservoir. Also, the removal of fluid from the portal reservoir may be effected in the reverse manner by suctioning the fluid in the portal reservoir through a right angle needle inserter, by applying suction to the catheter connected to the horizontal portion of the right angle needle while the vertical portion of the right angle needle is in fluid communication with the portal reservoir.

## Claims

1. Apparatus comprising:
an infuser (60) having a body (62) of a given shape, a blunt cannula (64) extending downwardly from a lower surface of the body (62) and a tube (66) connected to the body (64) for establishing a fluid communication path between said tube (66) and said blunt cannula (64);
a base (4) having a distal portion (6) and a proximal portion (8), having a configuration that form fits over the given shape of the body (62) of said infuser (60), an arm (12) having a second end (16) hingedly and movably connected to the distal portion of said base, a sharp cannula (24) attached to a first end (14) of said arm, a bore (42) defined between an opening (44a) at an upper surface and another opening (44) at a lower surface at the proximal portion of said base (4), the proximal portion of said base form fitting over the body (62) of said infuser (60) when said sharp cannula (24) is removably mated to said blunt cannula (64) through said bore at the proximal portion of said base and the tip (26) of said sharp cannula (24) exposed beyond the tip (64a) of said blunt cannula (64) with said arm (12) lying substantially longitudinally along said base (4);
wherein said sharp cannula is removed from said blunt cannula when said first end of said arm is moved pivotally in a direction away from said base into a second position.

2. An apparatus of claim 1, wherein in said second position of said arm (12), said sharp cannula (24) is retracted from said blunt cannula (64), and the tip of said sharp cannula (24) is positioned within said bore (42), said base (4) being separable from said infuser (60) after said sharp cannula (24) is retracted from said infuser (60).

3. An apparatus of claim 1, wherein said base (4) comprises at its distal portion (6) one part of a lock mechanism (18a, 18b) 52, 54) and wherein said arm (12) comprises at its second end (16) other part of said lock mechanism (28, 34) (50, 16a), said one and other parts coacting to prevent further movement of said arm (12) relative to said base (4) when said sharp cannula (24) is retracted from said infuser (60).

4. An apparatus of claim 1, further comprising a septum (68) provided on the body (62) of said infuser (60) through which said sharp cannula (24) passes when said sharp cannula (24) is mated to said blunt cannula (64), said septum sealing the body (62) and said infuser (60) when said sharp cannula (24) is removed from said infuser (60).

5. An apparatus of claim 1, wherein said infuser (60) comprises a body (62) having an aperture (68) at its top in longitudinal alignment with said blunt cannula (64), a septum at said aperture (68) to seal said body (62), wherein said sharp cannula (24) mates to said blunt cannula (64) through said septumed aperture (68), and wherein said infuser (68) has a lower surface adapted, in use, to lie substantially flat against the skin of a patient where the blunt cannula (64) is inserted to the patient.

6. An apparatus of claim 1, wherein the body (62) of said infuser (60 is dome shaped and wherein the recess in the lower surface of the proximal portion (8) of said base (6) is of complementary dome shape (72) such that it form fittedly covers said infuser (60) when said sharp cannula (24) is mated to said blunt cannula (64).

7. An apparatus of claim 1, further comprising at least one wing (82) provided at said infuser (60) to enable a user to maintain said infuser (60) against the skin of the patient as said sharp (24) cannula is removed from said blunt cannula (64) or when said base (4) is separated from said infuser (60).

8. An apparatus of claim 1, further comprising a port (78) which, in use, is implanted in the patient, said port(78) being pierceable by said sharp cannula (24) to position at least a portion of said blunt cannula (64) in said port (78), the portion of said blunt cannula (64) remaining inside said port (78) after the removal of said sharp cannula (24) from said blunt cannula (64) so that a fluid path is established between said port (78) and said tube (66) through said infuser (60).

9. A method of establishing a fluid path between a fluid store (70) and a port (78) implanted in a patient, comprising the steps of:
(a) providing an infuser having a body (62) of a given shape and a blunt cannula (64) extending downwardly therefrom and a tube (66) having one end connected thereto and another end connected to said fluid store, said tube being in fluid communication with said blunt cannula (64);
(b) providing a base (4) having a sharp cannula (24) and a proximal portion (8) having a configuration that has a shape that form fits over the body of said infuser;
(c) form fitting the proximal portion of said base (4) over the body of said infuser (60) and removably mating said sharp cannula to said blunt cannula until the tip (26) of said sharp cannula is exposed beyond the tip (64a) of said blunt cannula;
(d) inserting the combined sharp and blunt cannulas to the patient (74) to position said cannulas (24, 64) into said port (78); and
(e) removing said sharp cannula (24) from said blunt cannula (64) while leaving said blunt cannula (64) in said port; whereby a fluid communication path is established between said port and said fluid store through said blunt cannula (64) and said infuser.

10. A method of claim 9, wherein said base further comprises a distal portion (6), said step b further comprising the steps of:
hingedly connecting a second end (16) of an arm (12) to the distal portion of said base;
attaching said sharp cannula to a first end (14) of said arm;
providing a bore (42) at the proximal portion (8) of said body (4) through which said sharp cannula is removably mated to said blunt cannula (64), said bore (42) defined between an opening (44a) at an upper surface and another opening (44) at a lower surface of the proximal portion (8) of said base (4).

11. A method of claim 10, wherein said step e further comprises the step of:
maintaining the tip (26) of said sharp cannula (24) within said bore (42) once said sharp cannula (24) is removed from said infuser.

12. A method of claim 11, further comprising the step of:
providing respective coacting parts (18a, 18b & 28, 34) (52, 54 & 50, 16a) of a locking mechanism at the distal portion (6) of said body (4) and the second end (16) of said arm (12) to prevent said arm and said body from further movement relative to each other once the tip of said cannula is positioned within said bore.

13. A method of claim 9, further comprising the steps of:
providing an aperture (68) on the top of the body of said infuser in longitudinal alignment with said blunt cannula (64), said sharp cannula (24) mating to said blunt cannula (64) through said aperture (68);
adding a septum to said aperture to seal the body of said infuser after said sharp cannula is removed from said infuser.

14. A method of claim 13, further comprising the steps of:
configuring the proximal portion (72) of said base to cover said housing; and configuring the distal portion (74) of said base to cover said tube.

15. An apparatus of claim 1, wherein said infuser comprises an infuser body (62) adaptable for storing fluids, the blunt cannula (64) extending downwardly from said body adapted to be inserted into a port (78), and a tube (66) connected to a side of said body, a fluid path being established between said tube and said blunt cannula (64) through said body.

16. An apparatus of claim 15, further comprising a septum (68) provided at the top of said body in axial alignment with said blunt cannula, said septum adaptable to enable a sharp cannula (24) to be inserted therethrough to mate axially within said blunt cannula (64), with the tip (26) of said sharp cannula (24) protruding from the tip (64a) of said blunt cannula (64), said septum self sealing once said sharp cannula (24) is removed therefrom to maintain the fluid path between said blunt cannula (64) and said tubing as a closed fluid path.

17. An apparatus of claim 1, wherein the proximal portion of said base comprises a window (80) to enable a user to confirm that the proximal portion is appropriately form fitted over the body of said infuser.

## Patentansprüche

1. Vorrichtung, die Folgendes umfasst:
eine Infusionsvorrichtung (60) mit einem Körper (62) mit einer festgelegten Form, einer stumpfen Kanüle (64), die sich von einer unteren Fläche des Körpers (62) nach unten erstreckt, und einem Rohr (66), das zur Herstellung eines Flüssigkeitsverbindungswegs zwischen dem Rohr (66) und der stumpfen Kanüle (64) mit dem Körper (64) verbunden ist,
eine Basis (4) mit einem distalen Abschnitt (6) und einem proximalen Abschnitt (8), der eine Konfiguration aufweist, die formschlüssig über der festgelegten Form des Körpers (62) der Infusionsvorrichtung (60) aufsitzt, einem Arm (12) mit einem zweiten Ende (16), das drehbar und beweglich mit dem distalen Abschnitt der Basis verbunden ist, einer scharfen Kanüle (24), die an einem ersten Ende (14) des Arms angebracht ist, einer Bohrung (42), die zwischen einer Öffnung (44a) an einer oberen Fläche und einer anderen Öffnung (44) an einer unteren Fläche an dem proximalen Abschnitt der Basis (4) definiert ist, wobei der proximale Abschnitt der Basis formschlüssig über dem Körper (62) der Infusionsvorrichtung (60) aufsitzt, wenn die scharfe Kanüle (24) durch die Bohrung an dem proximalen Abschnitt der Basis herausnehmbar in die stumpfe Kanüle (64) eingepasst wird und die Spitze (26) der scharfen Kanüle (24) über die Spitze (64a) der stumpfen Kanüle (64) hinaus freiliegt, wobei der Arm (12) im Wesentlichen in Längsrichtung entlang der Basis (4) liegt;
wobei die scharfe Kanüle aus der stumpfen Kanüle herausgenommen wird, wenn das erste Ende des Arms drehbar in einer Richtung von der Basis weg in eine zweite Position bewegt wird.

2. Vorrichtung nach Anspruch 1, wobei die scharfe Kanüle (24) in der zweiten Position des Arms (12) aus der stumpfen Kanüle (64) zurückgezogen wird und die Spitze der scharfen Kanüle (24) in der Bohrung (42) positioniert ist, wobei die Basis (4) von der Infusionsvorrichtung (60) getrennt werden kann, nachdem die scharfe Kanüle (24) aus der Infusionsvorrichtung (60) zurückgezogen wurde.

3. Vorrichtung nach Anspruch 1, wobei die Basis (4) an ihrem distalen Abschnitt (6) einen Teil eines Arretiermechanismus (18a, 18b) (52, 54) umfasst und wobei der Arm (12) an seinem zweiten Ende (16) einen anderen Teil des Arretiermechanismus (28, 34) (50, 16a) umfasst, wobei der eine und der andere Teil zusammenwirken, um eine weitere Bewegung des Arms (12) in Bezug auf die Basis (4) zu verhindern, wenn die scharfe Kanüle (24) aus der Infusionsvorrichtung (60) zurückgezogen wird.

4. Vorrichtung nach Anspruch 1, die weiterhin eine Scheidewand (68) umfasst, die an dem Körper (62) der Infusionsvorrichtung (60) vorgesehen ist und durch die die scharfe Kanüle (24) hindurchgeht, wenn die scharfe Kanüle (24) in die stumpfe Kanüle (64) eingepasst wird, wobei die Scheidewand den Körper (62) und die Infusionsvorrichtung (60) abdichtet, wenn die scharfe Kanüle (24) aus der Infusionsvorrichtung (60) herausgenommen wird.

5. Vorrichtung nach Anspruch 1, wobei die Infusionsvorrichtung (60) einen Körper (62) umfasst, der einen Durchlass (68) auf seiner Oberseite in Längsausrichtung mit der stumpfen Kanüle (64) und eine Scheidewand an dem Durchlass (68) zum Abdichten des Körpers (62) aufweist, wobei die scharfe Kanüle (24) durch den mit einer Scheidewand versehenen Durchlass (68) in die stumpfe Kanüle (64) eingepasst wird, und wobei die Infusionsvorrichtung (68) eine untere Fläche aufweist, die im Gebrauch dazu eingerichtet ist, im Wesentlichen eben auf der Haut eines Patienten zu liegen, wo die stumpfe Kanüle (64) in den Patienten eingeführt ist.

6. Vorrichtung nach Anspruch 1, wobei der Körper (62) der Infusionsvorrichtung (60) domförmig ist und wobei die Aussparung in der unteren Fläche des proximalen Abschnitts (8) der Basis (6) eine komplementäre Domform (72) aufweist, so dass sie die Infusionsvorrichtung (60) formschlüssig abdeckt, wenn die scharfe Kanüle (24) in die stumpfe Kanüle (64) eingepasst wird.

7. Vorrichtung nach Anspruch 1, die weiterhin mindestens einen Flügel (82) umfasst, der an der Infusionsvorrichtung (60) vorgesehen ist, um einem Benutzer zu ermöglichen, die Infusionsvorrichtung (60) auf der Haut des Patienten zu halten, während die scharfe Kanüle (24) aus der stumpfen Kanüle (64) herausgenommen wird oder wenn die Basis (4) von der Infusionsvorrichtung (60) getrennt wird.

8. Vorrichtung nach Anspruch 1, die weiterhin einen Anschluss (78) umfasst, der im Gebrauch in den Patienten implantiert ist, wobei der Anschluss (78) von der scharfen Kanüle (24) durchstochen werden kann, um mindestens einen Abschnitt der stumpfen Kanüle (64) in dem Anschluss (78) zu positionieren, wobei der Abschnitt der stumpfen Kanüle (64) nach dem Herausnehmen der scharfen Kanüle (24) aus der stumpfen Kanüle (64) in dem Anschluss (78) bleibt, so dass ein Flüssigkeitsweg zwischen dem Anschluss (78) und dem Rohr (66) durch die Infusionsvorrichtung (60) hergestellt wird.

9. Verfahren zur Herstellung eines Flüssigkeitswegs zwischen einem Flüssigkeitsspeicher (70) und einem Anschluss (78), der in einen Patienten implantiert ist, wobei das Verfahren die folgenden Schritte umfasst:
(a) Vorsehen einer Infusionsvorrichtung mit einem Körper (62) mit einer festgelegten Form und einer stumpfen Kanüle (64), die sich von diesem nach unten erstreckt, und einem Rohr (66), das ein mit diesem verbundenes Ende und ein mit dem Flüssigkeitsspeicher verbundenes Ende aufweist, wobei das Rohr in Flüssigkeitsverbindung mit der stumpfen Kanüle (64) steht;
(b) Vorsehen einer Basis (4) mit einer scharfen Kanüle (24) und einem proximalen Abschnitt (8), der eine Konfiguration aufweist, die eine Form aufweist, die formschlüssig über dem Körper der Infusionsvorrichtung aufsitzt;
(c) formschlüssiges Aufsetzen des proximalen Abschnitts der Basis (4) über den Körper der Infusionsvorrichtung (60) und herausnehmbares Einpassen der scharfen Kanüle in die stumpfe Kanüle, bis die Spitze (26) der scharfen Kanüle über die Spitze (64a) der stumpfen Kanüle hinaus freiliegt;
(d) Einbringen der Kombination der scharfen Kanüle und der stumpfen Kanüle in den Patienten (74), um die Kanülen (24, 64) in dem Anschluss (78) zu positionieren; und
(e) Herausnehmen der scharfen Kanüle (24) aus der stumpfen Kanüle (64), während die stumpfe Kanüle (64) in dem Anschluss belassen wird;
wodurch ein Flüssigkeitsverbindungsweg zwischen dem Anschluss und dem Flüssigkeitsspeicher durch die stumpfe Kanüle (64) und die Infusionsvorrichtung hergestellt wird.

10. Verfahren nach Anspruch 9, wobei die Basis weiterhin einen distalen Abschnitt (6) umfasst, wobei der Schritt b weiterhin die folgenden Schritte umfasst:
drehbares Verbinden eines zweiten Endes (16) eines Arms (12) mit dem distalen Abschnitt der Basis;
Anbringen der scharfen Kanüle an einem ersten Ende (14) des Arms;
Vorsehen einer Bohrung (42) an dem proximalen Abschnitt (8) des Körpers (4), durch die die scharfe Kanüle herausnehmbar in die stumpfe Kanüle (64) eingepasst wird, wobei die Bohrung (42) zwischen einer Öffnung (44a) an einer oberen Fläche und einer anderen Öffnung (44) an einer unteren Fläche des proximalen Abschnitts (8) der Basis (4) definiert ist.

11. Verfahren nach Anspruch 10, wobei der Schritt e weiterhin den folgenden Schritt umfasst:
Halten der Spitze (26) der scharfen Kanüle (24) in der Bohrung (42), sobald die scharfe Kanüle (24) aus der Infusionsvorrichtung herausgenommen wurde.

12. Verfahren nach Anspruch 11, das weiterhin den folgenden Schritt umfasst:
Vorsehen jeweiliger zusammenwirkender Teile (18a, 18b & 28, 34) (52, 54 & 50, 16a) eines Arretiermechanismus an dem distalen Abschnitt (6) des Körpers (4) und des zweiten Endes (16) des Arms (12), um den Arm und den Körper an einer weiteren Bewegung in Bezug auf einander zu hindern, sobald die Spitze der Kanüle in der Bohrung positioniert wurde.

13. Verfahren nach Anspruch 9, das weiterhin die folgenden Schritte umfasst:
Vorsehen eines Durchlasses (68) auf der Oberseite des Körpers der Infusionsvorrichtung in Längsausrichtung mit der stumpfen Kanüle (64), wobei die scharfe Kanüle (24) durch den Durchlass (68) in die stumpfe Kanüle (64) eingepasst wird;
Hinzufügen einer Scheidewand zu dem Durchlass, um den Körper der Infusionsvorrichtung abzudichten, nachdem die scharfe Kanüle aus der Infusionsvorrichtung herausgenommen wurde.

14. Verfahren nach Anspruch 13, das weiterhin die folgenden Schritte umfasst:
Konfigurieren des proximalen Abschnitts (72) der Basis, um das Gehäuse abzudecken ; und Konfigurieren des distalen Abschnitts (74) der Basis, um das Rohr abzudecken.

15. Vorrichtung nach Anspruch 1, wobei die Infusionsvorrichtung einen Infusionsvorrichtungskörper (62), der zur Speicherung von Flüssigkeiten eingerichtet werden kann, die stumpfe Kanüle (64), die sich von dem Körper nach unten erstreckt und dazu eingerichtet ist, in einen Anschluss (78) eingeführt zu werden, und ein Rohr (66), das mit einer Seite des Körpers verbunden ist, umfasst, wobei ein Flüssigkeitsweg zwischen dem Rohr und der stumpfen Kanüle (64) durch den Körper hergestellt wird.

16. Vorrichtung nach Anspruch 15, die weiterhin eine Scheidewand (68) umfasst, die auf der Oberseite des Körpers in axialer Ausrichtung mit der stumpfen Kanüle vorgesehen ist, wobei die Scheidewand dazu eingerichtet werden kann, zu ermöglichen, eine scharfe Kanüle (24) dort hindurch eingeführt zu werden, um sie axial in die stumpfe Kanüle (64) einzupassen, wobei die Spitze (26) der scharfen Kanüle (24) aus der Spitze (64a) der stumpfen Kanüle (64) hervorragt, wobei die Scheidewand sich selbst abdichtet, sobald die scharfe Kanüle (24) daraus herausgenommen wurde, um den Flüssigkeitsweg zwischen der stumpfen Kanüle (64) und der Rohrleitung als einen geschlossenen Flüssigkeitsweg aufrechtzuerhalten.

17. Vorrichtung nach Anspruch 1, wobei der proximale Abschnitt der Basis ein Fenster (80) umfasst, um einem Benutzer zu ermöglichen, zu bestätigen, dass der proximale Abschnitt auf geeignete Weise formschlüssig über den Körper der Infusionsvorrichtung aufgesetzt ist.

## Revendications

1. Appareil comprenant :
un perfuseur (60) ayant un corps (62) d'une forme donnée, une canule mousse (64) s'étendant vers le bas depuis une surface inférieure du corps (62) et un tube (66) rattaché au corps (64) pour établir un chemin de communication fluidique entre ledit tube (66) et ladite canule mousse (64) ;
une base (4) ayant une partie distale (6) et une partie proximale (8), ayant une configuration qui s'ajuste de par sa forme au-dessus de la forme donnée du corps (62) dudit perfuseur (60), un bras (12) ayant une deuxième extrémité (16) rattachée de manière articulée et mobile à la partie distale de ladite base, une canule acérée (24) attachée à une première extrémité (14) dudit bras, un alésage (42) défini entre une ouverture (44a) à une surface supérieure et une autre ouverture (44) à une surface inférieure à la partie proximale de ladite base (4), la partie proximale de ladite base s'ajustant de par sa forme au-dessus du corps (62) dudit perfuseur (60) lorsque ladite canule acérée (24) est accouplée de manière amovible à ladite canule mousse (64) à travers ledit alésage à la partie proximale de ladite base et le bout (26) de ladite canule acérée (24) est exposé au-delà du bout (64a) de ladite canule mousse (64) avec ledit bras (12) reposant essentiellement longitudinalement le long de ladite base (4) ;
dans lequel ladite canule acérée est enlevée de ladite canule mousse lorsque ladite première extrémité dudit bras est déplacée de manière pivotante dans une direction l'écartant de la dite base pour la mettre dans une deuxième position.

2. Appareil selon la revendication 1, dans lequel, dans ladite deuxième position dudit bras (12), ladite canule acérée (24) est rétractée de ladite canule mousse (64), et le bout de ladite canule acérée (24) est positionné à l'intérieur dudit alésage (42), ladite base (4) pouvant être séparée dudit perfuseur (60) après que ladite canule acérée (24) a été rétractée dudit perfuseur (60).

3. Appareil selon la revendication 1, dans lequel ladite base (4) comprend à sa partie distale (6) une partie d'un mécanisme de blocage (18a, 18b) (52, 54), et dans lequel ledit bras (12) comprend à sa deuxième extrémité (16) l'autre partie dudit mécanisme de blocage (28, 34) (50, 16a), lesdites une et autre parties agissant ensemble pour empêcher tout mouvement supplémentaire dudit bras (12) par rapport à ladite base (4) lorsque ladite canule acérée (24) est rétractée dudit perfuseur (60).

4. Appareil selon la revendication 1, comprenant en outre un septum (68) prévu sur le corps (62) dudit perfuseur (60) à travers lequel ladite canule acérée (24) passe lorsque ladite canule acérée (24) est accouplée à ladite canule mousse (64), ledit septum scellant le corps (62) et ledit perfuseur (60) lorsque ladite canule acérée (24) est enlevée dudit perfuseur (60).

5. Appareil selon la revendication 1, dans lequel ledit perfuseur (60) comprend un corps (62) ayant une ouverture (68) à son sommet en alignement longitudinal avec ladite canule mousse (64), un septum à ladite ouverture (68) pour sceller ledit corps (62), dans lequel ladite canule acérée (24) s'accouple à ladite canule mousse (64) à travers ladite ouverture à septum (68), et dans lequel ledit perfuseur (68) a une surface inférieure adaptée de façon à reposer, en cours d'utilisation, à plat contre la peau d'un patient là où la canule mousse (64) est insérée dans le patient.

6. Appareil selon la revendication 1, dans lequel le corps (62) dudit perfuseur (60) a la forme d'un dôme et dans lequel l'évidement dans la surface inférieure de la partie proximale (8) de ladite base (6) a une forme de dôme complémentaire (72) de manière à s'ajuster de par sa forme au-dessus dudit perfuseur (60) lorsque ladite canule acérée (24) est accouplée à ladite canule mousse (64).

7. Appareil selon la revendication 1, comprenant en outre au moins une aile (82) prévue au niveau dudit perfuseur (60) pour permettre à un utilisateur de maintenir ledit perfuseur (60) contre la peau du patient tandis que ladite canule acérée (24) est enlevée de ladite canule mousse (64) ou lorsque ladite base (4) est séparée dudit perfuseur (60).

8. Appareil selon la revendication 1, comprenant en outre un orifice (78) qui, en cours d'utilisation, est implanté dans le patient, ledit orifice (78) pouvant être percé par ladite canule acérée (24) afin de positionner au moins une partie de ladite canule mousse (64) dans ledit orifice (78), la partie de ladite canule mousse (64) restant à l'intérieur dudit orifice (78) après l'enlèvement de ladite canule acérée (24) de ladite canule mousse (64) de manière à ce qu'un chemin de fluide soit établi entre ledit orifice (78) et ledit tube (66) à travers ledit perfuseur (60).

9. Procédé pour établir un chemin de fluide entre une réserve de fluide (70) et un orifice (78) implanté dans un patient, comprenant les étapes consistant à :
(a) prévoir un perfuseur ayant un corps (62) d'une forme donnée et une canule mousse (64) s'étendant vers le bas depuis celui-ci et un tube (66) ayant une extrémité rattachée à celui-ci et une autre extrémité rattachée à ladite réserve de fluide, ledit tube étant en communication fluidique avec ladite canule mousse (64) ;
(b) prévoir une base (4) ayant une canule acérée (24) et une partie proximale (8) ayant une configuration qui a une forme qui s'ajuste au-dessus du corps dudit perfuseur ;
(c) ajuster de par sa forme la partie proximale de ladite base (4) au-dessus du corps dudit perfuseur (60) et à accoupler de manière amovible ladite canule acérée à ladite canule mousse jusqu'à ce que le bout (26) de ladite canule acérée soit exposé au-delà du bout (64a) de ladite canule mousse ;
(d) insérer les canules acérée et mousse combinées dans le patient (74) afin de positionner lesdites canules (24, 64) dans ledit orifice (78) ; et à
(e) enlever ladite canule acérée (24) de ladite canule mousse (64) tout en laissant ladite canule mousse (64) dans ledit orifice ; ce qui fait qu'un chemin de communication fluidique est établi entre ledit orifice et ladite réserve de fluide à travers ladite canule mousse (64) et ledit perfuseur.

10. Procédé selon la revendication 9, dans lequel ladite base comprend en outre une partie distale (6), ladite étape b comprenant en outre les étapes consistant à :
rattacher de manière articulée une deuxième extrémité (16) d'un bras (12) à la partie distale de ladite base ;
attacher ladite canule acérée à une première extrémité (14) dudit bras ;
prévoir un alésage (42) à la partie proximale (8) dudit corps (4) à travers lequel ladite canule acérée est accouplée de manière amovible à ladite canule mousse (64), ledit alésage (42) étant défini entre une ouverture (44a) à une surface supérieure et une autre ouverture (44) à une surface inférieure de la partie proximale (8) de ladite base (4).

11. Procédé selon la revendication 10, dans lequel ladite étape e comprend en outre l'étape consistant à :
maintenir le bout (26) de ladite canule acérée (24) à l'intérieur dudit alésage (42) une fois que ladite canule acérée (24) a été enlevée dudit perfuseur.

12. Procédé selon la revendication 11, comprenant en outre l'étape consistant à :
prévoir des parties respectives agissant ensemble (18a, 18b & 28, 34) (52, 54 & 50, 16a) d'un mécanisme de blocage à la partie distale (6) dudit corps (4) et à la deuxième extrémité (16) dudit bras (12) afin d'empêcher ledit bras et ledit corps de continuer de bouger l'un par rapport à l'autre une fois que le bout de ladite canule est positionné à l'intérieur dudit alésage.

13. Procédé selon la revendication 9, comprenant en outre les étapes consistant à :
prévoir une ouverture (68) sur le sommet du corps dudit perfuseur en alignement longitudinal avec ladite canule mousse (64), ladite canule acérée (24) s'accouplant à ladite canule mousse (64) à travers ladite ouverture (68) ;
ajouter un septum à ladite ouverture de façon à sceller le corps dudit perfuseur après que ladite canule acérée a été enlevée dudit perfuseur.

14. Procédé selon la revendication 13, comprenant en outre les étapes consistant à :
configurer la partie proximale (72) de ladite base de façon à couvrir ledit boîtier ; et à
configurer la partie distale (74) de ladite base pour couvrir ledit tube.

15. Appareil selon la revendication 1, dans lequel ledit perfuseur comprend un corps de perfuseur (62) adaptable pour stocker des fluides, la canule mousse (64) s'étendant vers le bas depuis ledit corps étant adaptée de façon à être insérée dans un orifice (78), et un tube (66) rattaché à un côté dudit corps, un chemin de fluide étant établi entre ledit tube et ladite canule mousse (64) à travers ledit corps.

16. Appareil selon la revendication 15, comprenant en outre un septum (68) prévu au sommet dudit corps en alignement axial avec ladite canule mousse, ledit septum étant adaptable de façon à permettre à une canule acérée (24) d'être insérée à travers lui de façon à s'accoupler axialement à l'intérieur de ladite canule mousse (64), avec le bout (26) de ladite canule acérée (24) faisant saillie du bout (64a) de ladite canule mousse (64), ledit septum se scellant une fois que ladite canule acérée (24) est enlevée de celui-ci afin de maintenir le chemin de fluide entre ladite canule mousse (64) et ladite tubulure comme un chemin de fluide fermé.

17. Appareil selon la revendication 1, dans lequel la partie proximale de ladite base comprend une fenêtre (80) pour permettre à un utilisateur de confirmer que la partie proximale est ajustée de par sa forme de manière appropriée au-dessus du corps dudit perfuseur.
